# EUROPEAN PATENT SPECIFICATION

(11) **EP 1 689 293 B1**
(45) Date of publication and mention of the grant of the patent: **03.12.2008**
(21) Application number: 04819573.9
(22) Date of filing: 03.12.2004
(51) Int. Cl.: A61B 5/00

(54) **MEANS FOR TREATING DIZZINESS AND BALANCE DISTURBANCES**
MITTEL ZUR BEHANDLUNG VON SCHWINDEL UND GLEICHGEWICHTSSTÖRUNGEN
MOYENS DE TRAITEMENT DU VERTIGE ET DES TROUBLES DE L'EQUILIBRE

(30) Priority: 05.12.2003 CH 208203; 03.02.2004 CH 15804
(43) Date of publication of application: 16.08.2006
(73) Proprietor: Weluga-Pharm Anstalt, 9485 Nendeln (LI)
(72) Inventor: EHRENBERGER, Klaus, A-1090 Vienna (AT); THURNER, Stefan, A-1080 Vienna (AT)
(74) Representative: Hasler, Erich
(86) International application number: PCT/CH2004/000722
(87) International publication number: WO 2005/053522

(56) References cited:
- EP-A- 0 411 821
- US-B1- 6 213 956
- US-B1- 6 540 355
- RIKE FOCHLER: "Antisturztraining" MODERN TIMES, [Online] 5 December 2003 (2003-12-05), XP002280982 Retrieved from the Internet: URL:http://moderntimes.orf.at/> [retrieved on 2004-05-19]
- SHIMIZU YU ET AL: "Multifractal spectra as a measure of complexity in human posture" FRACTALS WORLD SCIENTIFIC SINGAPORE, vol. 10, no. 1, March 2002 (2002-03), pages 103-116, XP008043243 ISSN: 0218-348X

## Description

### Field of the invention

The invention relates to a means for treating dizziness and balance disturbances.

### Introduction

65 % of people over 60 years of age complain of dizziness or poor balance and troubles associated therewith. In Austria the proportion of people over 60 is 21 % (1.7 million people). This corresponds to about 1.1 million people who are affected by dizziness or poor balance. Age-related dizziness complaints are a manifestation of a gradually decreasing functionality of the orientation system. The intact biological function of spatial orientation is characterised by a complex interaction between numerous control mechanisms which register the space (primarily balance organs, eyes, somato sensory system) and which purposefully control the musculature of the motor control system so an upright posture and movement in space counter to gravity and other acceleration forces is possible. Complex functions guarantee a rapid reaction in numerous degrees of freedom and this makes it possible for the individual to react quickly and purposefully to unexpected environmental influences. Speed and dynamics of complex functions generally decrease with age. The aging phenomenon also affects the orientation system with the above-described, clinically manifest, functional effects. Consistent training counteracts the consequences of ageing processes on complex biological systems (for example cardiovascular training, swallow-reflex training, etc). This also applies to the orientation system. Exercise for the elderly and apparatus-assisted stability-promoting movement exercises primarily use the somatosensory control input for targeted system training. However, these training concepts are opposed by age-related joint problems, muscular atrophy and in many case lack of motivation, so fall-induced injuries among older people increase as a function of the increasing age of society.

US 6,540,355 relates to a method, a device and associated software for exercising the human eye with a monitor, on which a plurality of objects are projected in such a way that portions of the objects have a contrast which changes at a rate of two or fewer oscillations per second. The objects preferably consist of triangles, rectangles or lines with contrasting colours, for example black/white, red/green, etc., arranged in pairs. Preferably, a large number of symbols consisting of a plurality of lines arranged parallel and side by side is statically projected onto the monitor. The lines of the symbols are - with the exception of one line - of the same length. The vision efficiency may then be calculated from the number of correctly recognised symbols. The described method is used to test the power of vision of an individual directly at the computer workstation. This allows, for example, the adjustments of the monitor to be adapted to the user. The exercises are also used to reduce the fatigue of the eyes. A characteristic of the method of US 6,540,355 is that the projected images are static, i.e. do not move on the monitor. Another piece of prior art is the article "Multifractal spectra as a measure of complexity in human posture" by Yu. Shimizu et al. in Fractals, vol. 10, no. 1, 2002.

### Object of the invention

The object of the present invention is therefore to provide a means for treating dizziness and balance disturbances. A further object is to provide a method of producing a means for treating dizziness and balance disturbances.

### Description

According to the invention the object is achieved whith a means according to claim 1 and a method according to claim 9. Advantageous configurations of the method are defined in the sub-claims. The means has the advantage that a movement structure is imposed on the observer, for example an older person in whom the movement structure has slowed down, as exists in healthy people. This is a completely new method for improving the balance capacity and of treating balance disturbances or dizziness, which frequently occur in older people, in particular. The means according to the invention is therefore a visual therapy for influencing physiological processes such as the orientation system by using the complex system.

Basically, the information existing as a sequence of images or stored information can be a sequence of images comprising a plurality of letters or words or different objects. This is of importance as it has to be ensured that the eyes of the patient follow the moving text or the moving objects. The tests carried out by the inventors have surprisingly shown that a single image, which is moved on a display, is not capable of training the movement pattern of the eye. It is not sufficient for the information to be stochastically moved to and fro and up and down on the display, rather it must be ensured that the observer follows the jerkily displaced information with his eyes. This may best be achieved in that the information is a text of a specific length, preferably a text comprising a plurality of lines. If the information exists as a readable text then this has the advantage that the reader is encouraged to follow information jumping around on the display if he wishes to register the text. At the same time a check is easily possible if the reader has to read the text out loud. Basically it is conceivable to simultaneously record the text when a microphone is provided. The recorded text allows the doctor to check whether the eye training has been carried out properly. It is also possible to carry out the training to improve the balance capacity at home.

The position of the text on the display is advantageously stochastically varied at such a rate that the text can still just be read by the observer. In practice the situation is such that the text appears at a first location during a first time period, at a second location, which is spaced apart from the first location, during a second subsequent time period, at a third location, which is spaced apart from the second location, during a third subsequent time period and so on, the direction of displacement between a location n (of period n) and a location n+1 (of period n+1) stochastically varying. In the process the displacement distance is in each case preferably also varied within a predetermined range.

The text is expediently varied at such a rate that it is only stationary for fractions of a second in each case. However, the standstill times should be sufficient for the observer to be able to perceive the information with the senses.

The time period of the standstill is in each case preferably between 10 and 2,000, preferably between 80 and 1,000 and most preferably between 80 and 800 milliseconds.

The means according to the invention can exist as a video sequence in a format that can be reproduced on a computer (*.mpeg, *.avi, *.wmf, etc.). This has the advantage that the video sequence can be electronically transmitted via the Internet. However, the means according to the invention can be stored on a data carrier (disk, CD-ROM, magnetic tape). It is also conceivable, however, that only one program is available which stochastically varies the position on the display of any desired text which can be determined by the user in the manner according to the invention.

According to a further aspect of the invention, a method is claimed for producing a means according to any one of claims 1 to 8, which is characterised by the features of claim 9. The program can basically be made available to the user so he can select any desired text with which he wishes to carry out the eye training. The program can also be used to displace the information in any desired directions in a time sequence and at random displacement distances within a certain range and to record the generated pattern on a data carrier, for example a video cassette, DVD or the like, as a sequence of images (video sequence).

In a therapeutic method for treating dizziness and/or balance disturbances and/or for improving the balance capacity in older people, which does not form part of the invention, a piece of information which can reproduced on suitable reproduction equipment and can be visually registered and registered by the other senses, is stochastically displaced during reproduction on a display at such a rate that the information is stationary only for fractions of a second in each case. In the process, it must, however, still be possible for the observer's eye to register or read the information. According to a particularly preferred variation of the method, a text comprising a plurality of largely different words is stochastically moved on a display. In this case the patient has to attempt to read the text out loud. The eye training can be checked by the patient himself or by a third party by the process of reading out loud. It is also conceivable to record the voice of the patient so a subsequent check or a remote check is possible. The text displayed can be a one line or a multi-line text.

### Training of the orientation system via the visual check input:

Eye movements are a good reflection of the complex structure of the orientation system with its numerous degrees of freedom. The dynamics of the system corresponds to a non-linear system with a "self-similar" or "fractal" structure, of which the inherent functional order can be well depicted using the method of fractal geometry. This functional structure changes with age with alarming contraction of necessary degrees of freedom. According to the invention, it is accordingly proposed to re-program the "fractal" structure in the orientation system using non-linear eye movements and to thus return a "youthful" function to the system via eye training. The visual stimulation takes place via a text-image with a non-linear (stochastic) movement pattern which is projected onto a screen. The patient has to read the text out loud to ensure that the eyes are following the movement pattern. This training method is also reasonable for wearers of glasses and is completely independent of the condition of the movement apparatus of the body. It was possible to prove the high clinical value of this visual dizziness therapy in a "Proof of concept" study. The innovative core to this therapy is the movement pattern which is characterised as follows:

### The movement pattern

It was found from the movement patterns of the body movements when standing that the "degree of self-similarity" (fractality) decreases with age in these patterns. It is therefore proposed to impose "self-similar" patterns on the balance system again. The movement pattern is characterised as follows:
- The image/text moves in two dimensions. The movement of the centre of the image is described by x(t) and y(t) components, where t is the time. After its appearance, the position of the image/text remains constant for a period of Δt seconds (Δt is between 10 and 2,000, preferably between 80 and 1,000, and most preferably between 80 and 800 milliseconds). After this stationary period the position of the image is displaced to a new position. The displacement in the x direction is δₓ and δ_{y} in the y direction. The image remains at this position for Δt seconds again, is subsequently displaced again, etc.
- The movement pattern is characterised by the spectrum S(ω) of the movement components x and y (absolute square of the Fourier transform), where ω is the frequency. The spectrum follows (over at least half an order of magnitude of the frequency) a power law, i.e. S = cω^{-β}. Here c is any desired constant and β a real number between 0 and 4.
   This connection also applies to the increments of x and y, i.e. δₓ(t) = x(t) - x(t-Δt) and δ_{y}(t) = y(t) - y(t-Δt), (of course with different c and β). The term t-Δt represents the instant of the Δt seconds before t is or was, which results in absolute values from the specially selected time resolution or update rate of the system.
- The movement pattern is also characterised by the function F(τ) = {[x(t + τ) - x(t)]²}ₜ = dτ^{2H}, wherein d is any constant and H is the Hurst exponent which is between 0 and 1. {.}ₜ represents the average over time. This law should exist at least over half an order of magnitude in τ. The same applies to component y.
- Amplitudes (mean values and standard deviation of δₓ and δ_{y}) and rate (update rate or time increment Δt) are variable and individually adjusted.

In the literature δₓ and δ_{y} are sometimes called "correlated noise" or "anti-correlated noise" or "fractal noise" or "fractional Brownian noise".

### Embodiment

The therapy used in a study consisted of 10 half-hour sessions. The tests showed that a daily half-hour exercise drastically intensifies the improvement in patients suffering from dizziness and training effect in people with poor balance.

### Description of the enclosures

The accompanying graphics relate to the quantative measurements of the balance capacity in patients suffering from dizziness before and after treatment.
- Fig. A shows the rate of the balance capacity during six different tests which were each repeated three times. A balance factor (composite) is calculated from these measurements. This factor is a measure of the balance capacity. In healthy young people this quantity is greater than 70. Over the course of treatment this quantity could be drastically improved in patients (from approx. 60 to approx. 80).
- Fig. B shows the functionality of individual sensory inputs. SOM is the somatosensory input, VIS the visual input and VEST the vestibular input. The value is given as between 0 and 100 and shown in colour: poor = red, grey = normal case, green = good. In both patients the vestibular input before treatment is poor and can only be dramatically improved by visual training, however.

The disclosure relates to a means in the form of a piece of information like a text which can be reproduced on suitable reproduction equipment and can be visually registered, which information exists as a text, for example in the form of a video sequence, which, when reproduced on a display, shows the text being stochastically displaced at such a rate that the information can still just be registered by the observer's eye but stands still only for fractions of a second. The time period of the standstill is in each case advantageously between 8 and 150, preferably between 80 and 200, and most preferably between 80 and 150 milliseconds.

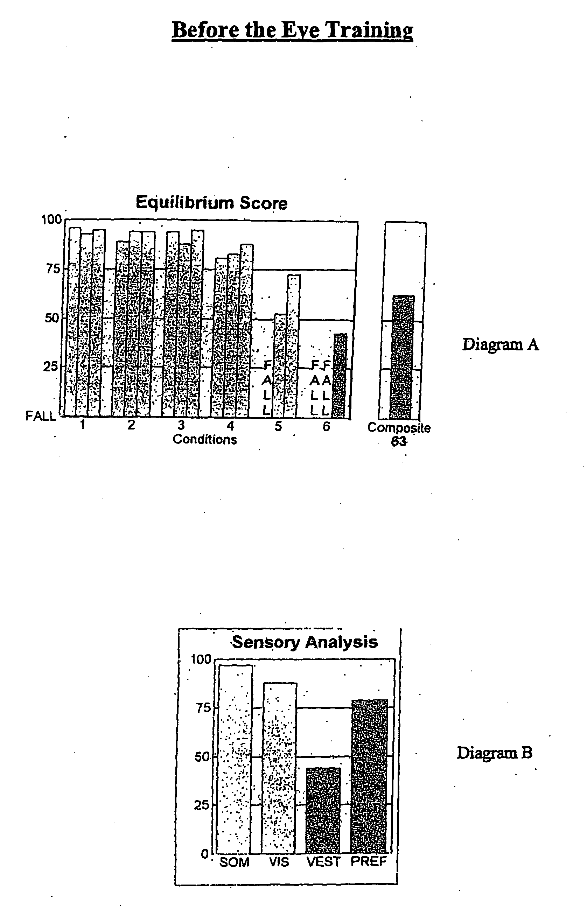

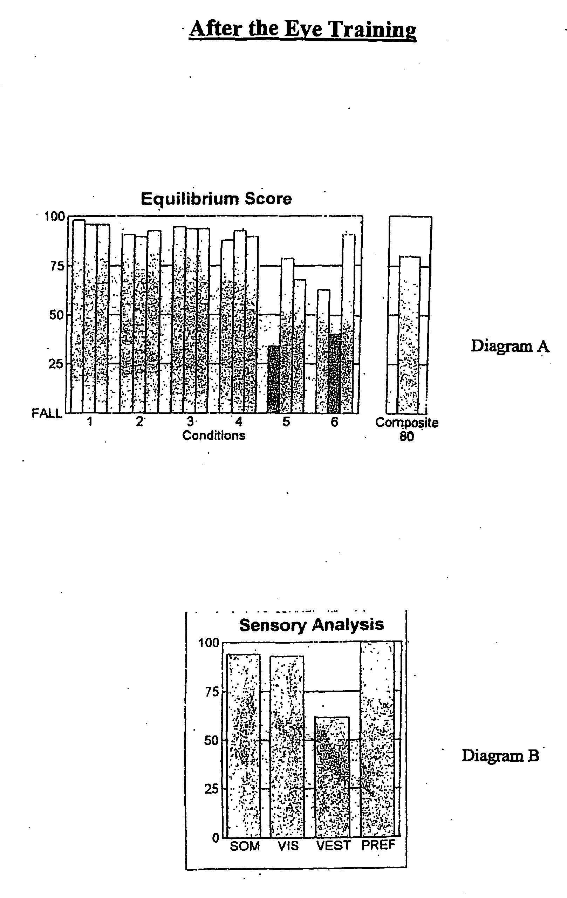

## Claims

1. Means for treating dizziness and/or balance disturbances and/or for improving the balance capacity in older people by stimulating the visual system stochastically, where the means is a data carrier with a text of a specific length stored thereon, which upon reproduction on a display by suitable reproduction equipment, is stochastically displaced on the display at such a rate that the information can still be registered by the observer's eye, such that the movement pattern of the text is **characterised by** the spectrum S(ω) of the movement components x and y (absolute square of the Fourier transform), where ω is the frequency and S(ω) follows a power law of the type S = cω^{-β}, wherein c is any constant and β is a real number between 0 and 4.

2. Means according to claim 1, **characterised in that** the text has a plurality of lines.

3. Means according to claim 2, **characterised in that** the position of the text on the display is varied in any desired direction at such a rate that the text can still just be read by the observer.

4. Means according to any one of claims 1 to 3, **characterised in that** the text on the display appears at a first location during a first time period, at a second location, which is spaced apart from the first location, during a second subsequent time period, at a third location, which is spaced apart from the second location, during a third subsequent time period and so on, the direction of displacement between a location n (of period n) and a location n+1 (of period n+1) randomly varying.

5. Means according to any one of claims 1 to 4, **characterised in that** the displacement distance is varied within a predetermined range.

6. Means according to any one of claims 1 to 5, **characterised in that** the text is varied at such a rate that the information is in each case stationary for only fractions of a second up to a maximum of two seconds.

7. Means according to any one of claims 1 to 6, **characterised in that** the time period of the standstill is in each case between 10 and 2,000, preferably between 80 and 1,000, and most preferably between 80 and 800 milliseconds.

8. Means according to claim 1, **characterised in that** the movement pattern is further **characterised by** the function F(τ)= {[x(t + τ)- x(t)]²}ₜ = dτ^{2H}, where d is any desired constant and H is the Hurst exponent which is between 0 and 1.

9. Method for producing a means in the form of a data carrier according to any one of claims 1 to 8, **characterised in that** a text of a specific length which can be depicted on a display is stochastically displaced on the display at such a rate that the information can still be registered by the observer's eye, using a program, such that the movement pattern of the text is **characterised by** the spectrum S(ω) of the movement components x and y (absolute square of the Fourier transform), where ω is the frequency and S(ω) follows a power law of the type S = cω^{-β}, wherein c is any constant and β is a real number between 0 and 4.

10. Method according to claim 9, **characterised in that** the movement pattern generated by the program is recorded on a further data carrier, for example a video cassette, DVD, memory stick or the like.

## Patentansprüche

1. Mittel zur Behandlung von Schwindel und/ oder Gleichgewichtsstörungen und/ oder zur Verbesserung der Balancefähigkeit bei älteren Menschen durch stochastische Stimulierung des visuellen Systems,
**dadurch gekennzeichnet,**
**dass** das Mittel ein Datenträger mit einem Text von spezifischer Länge ist, der darauf gespeichert ist, welcher Text bei der Reproduktion auf einem Display durch ein geeignetes Wiedergabegerät stochastisch in einer solchen Geschwindigkeit angezeigt wird, dass die Information durch das Auge des Betrachters noch registriert werden kann und, dass das Bewegungsmuster des Textes durch das Spektrum S(ω) der Bewegungskomponenten x und y (Absolutquadrat der Fouriertransformierten) charakterisiert ist, wobei ω die Frequenz und S(ω) einem Potenzgesetz der Art S = cω^{-β} folgt, wobei c eine beliebige Konstante, und β eine reelle Zahl zwischen 0 und 4 ist.

2. Mittel nach Anspruch 1, **dadurch gekennzeichnet, dass** der Text mehrere Zeilen aufweist.

3. Mittel nach Anspruch 2, **dadurch gekennzeichnet, dass** die Position des Textes auf dem Display in jeder gewünschten Richtung mit einer solchen Geschwindigkeit variiert wird, dass der Text durch den Betrachter gerade noch lesbar ist.

4. Mittel nach einem der Ansprüche 1 bis 3, **dadurch gekennzeichnet, dass** der Text auf dem Display während einer ersten Zeitperiode an einem ersten Ort erscheint, während einer zweiten darauffolgenden Zeitperiode an einem zweiten Ort erscheint, welcher vom ersten Ort beabstandet ist, während einer dritten darauffolgenden Zeitperiode an einem dritten Ort erscheint, welcher vom zweiten Ort beabstandet ist, und so fort, wobei die Verschieberichtung zwischen einem Ort n (der Periode n) und einem Ort n+1 (der Periode n+1) zufällig variiert.

5. Mittel nach einem der Ansprüche 1 bis 4, **dadurch gekennzeichnet, dass** die Verschiebedistanz in einem bestimmten Bereich variiert wird.

6. Mittel nach einem der Ansprüche 1 bis 5, **dadurch gekennzeichnet, dass** der Text mit einer solchen Geschwindigkeit variiert wird, dass die Information jeweils nur für Sekundenbruchteile bis maximal 2 Sekunden still steht.

7. Mittel nach einem der Ansprüche 1 bis 7, **dadurch gekennzeichnet, dass** die Zeitperiode des Stillstandes jeweils zwischen 10 und 2000, vorzugsweise zwischen 80 und 1000, und ganz besonders zwischen 80 und 800 Millisekunden beträgt.

8. Mittel nach Anspruch 1, **dadurch gekennzeichnet, dass** das Bewegungsmuster weiters charakterisiert ist durch die Funktion F(τ) = {[x(t+ τ)- x(t)]²}ₜ= dτ^{2H}, wobei d eine beliebige Konstante und H der Hurstexponent ist, der zwischen 0 und 1 liegt.

9. Verfahren zur Herstellung eines Mittels in Gestalt eines Datenträgers gemäss einem der Ansprüche 1 bis 8, **dadurch gekennzeichnet, dass** ein auf einem Display darstellbarer Text einer spezifischen Länge auf dem Display stochastisch mit Hilfe eines Programmes mit einer solchen Geschwindigkeit verschoben wird, dass die Information durch das Auge des Betrachters noch registriert werden kann und dass das Bewegungsmuster des Textes durch das Spektrum S(ω) der Bewegungskomponenten x und y (Absolutquadrat der Fouriertransformierten) charakterisiert ist, wobei ω die Frequenz und S(ω) einem Potenzgesetz der Art S = cω^{-β} folgt, wobei c eine beliebige Konstante, und β eine reelle Zahl zwischen 0 und 4 ist.

10. Verfahren nach Anspruch 9, **dadurch gekennzeichnet, dass** das durch das Programm generierte Bewegungsmuster auf einem weiteren Datenträger, z.B. einer Videokassette, DVD, memory stick oder dergleichen, aufgezeichnet wird.

## Revendications

1. Moyen pour traiter les étourdissements et/ou les troubles d'équilibre et/ou pour améliorer la capacité d'équilibre des personnes âgées en stimulant le système visuel de manière stochastique, le moyen étant un support de données avec un texte d'une longueur spécifique qui y est mémorisé qui, après reproduction sur un dispositif d'affichage par un équipement de reproduction approprié, est déplacé de manière stochastique sur le moyen d'affichage à une vitesse telle que l'information peut encore être enregistrée par l'oeil de la personne qui observe de telle manière que le schéma du mouvement du texte est **caractérisé par** le spectre S(ω) des composantes du mouvement x et y (carré absolu de la transformation de Fourier), dans lequel ω est la fréquence et S(ω) suite une loi de puissance du type S = cω^{-β}, dans lequel c est n'importe quelle constante et β est un nombre réel entre 0 et 4.

2. Moyen selon la revendication 1, **caractérisé en ce que** le texte a une pluralité de lignes.

3. Moyen selon la revendication 2, **caractérisé en ce que** la position du texte sur le dispositif d'affichage est variée dans n'importe quelle direction souhaitée à une vitesse telle que le texte peut encore tout juste lu par la personne qui observe.

4. Moyen selon l'une quelconque des revendications 1 à 3, **caractérisé en ce que** le texte sur le dispositif d'affichage apparaît à un premier endroit pendant une première période de temps, à un second endroit qui est espacé du premier endroit pendant une seconde période de temps qui suit, à un troisième endroit qui est espacé du second endroit pendant une troisième période de temps qui suit et ainsi de suite, la direction du déplacement entre un endroit n (de la période n) et un endroit n+1 (de la période n+1) variant de manière aléatoire.

5. Moyen selon l'une quelconque des revendications 1 à 4, **caractérisé en ce que** la distance de déplacement varie au sein d'une plage prédéfinie.

6. Moyen selon l'une quelconque des revendications 1 à 5, **caractérisé en ce que** le texte est varié à une vitesse telle que l'information est, dans chaque cas, stationnaire pour seulement des fractions de seconde jusqu'à un maximum de deux secondes.

7. Moyen selon l'une quelconque des revendications 1 à 6, **caractérisé en ce que** la période de temps de l'état stationnaire est, dans chaque cas, entre 10 et 2000, de préférence entre 80 et 1000 et de manière la plus préférée entre 80 et 800 millisecondes.

8. Moyen selon la revendication 1, **caractérisé en ce que** le schéma du mouvement est de plus **caractérisé par** la fonction F(_{T}) = {[x(t + T) - x(t)]²} = d_{T}^{2H} dans laquelle d est n'importe quelle constante souhaitée et H est l'exposant de Hurst qui est entre 0 et 1.

9. Procédé de production d'un moyen en forme de support de données selon l'une quelconque des revendications 1 à 8, **caractérisé en ce qu'**un texte d'une longueur spécifique qui peut être représenté sur un dispositif d'affichage est déplacé de manière stochastique sur le dispositif d'affichage à une vitesse telle que l'information peut encore être enregistrée par l'oeil de la personne qui observe, en utilisant un programme tel que le schéma du mouvement du texte est **caractérisé par** le spectre S(ω) des composantes de mouvement x et y (carré absolu de la transformation de Fourier) dans lequel ω est la fréquence et S(ω) suite une loi de puissance du type S = cω^{-β}, dans lequel c est n'importe quelle constante et β est un nombre réel entre 0 et 4.

10. Procédé selon la revendication 9, **caractérisé en ce que** le schéma du mouvement généré par le programme est enregistré sur un support de données supplémentaire, par exemple une cassette vidéo, un DVD, un stick mémoire ou équivalent.
